# EUROPEAN PATENT SPECIFICATION

(11) **EP 1 143 934 B1**
(45) Date of publication and mention of the grant of the patent: **04.06.2003**
(21) Application number: 00901411.9
(22) Date of filing: 13.01.2000
(51) Int. Cl.: A61K 9/16, A61K 38/19, A61K 39/39, A61K 9/51

(54) **GENETIC IMMUNIZATION WITH CO-DELIVERY OF NUCLEIC ACID AND CYTOKINES**
GENETISCHE IMMUNISIERUNG MIT GLEICHZEITIGER VERABREICHUNG VON NUKLEINSÄUREN UND ZYTOKINEN
IMMUNISATION GENETIQUE AVEC ADMINISTRATION CONJOINTE D'ACIDE NUCLEIQUE ET DE CYTOKINES

(30) Priority: 13.01.1999 US 115849 P; 15.01.1999 US 116242 P
(43) Date of publication of application: 17.10.2001
(73) Proprietor: Johns Hopkins University School of Medicine, Baltimore, MD 21202 (US)
(72) Inventor: LIU, Shu, Quin, Baltimore, MD 21211 (US); SONG, Ruijiang, Baltimore, MD 21205 (US); AUGUST, J., Thomas, Baltimore, MD 21210 (US); LEONG, Kam, W., Ellicott City, MD 21042 (US)
(74) Representative: Tombling, Adrian George
(86) International application number: US0000730
(87) International publication number: WO00041679

(56) References cited:
- WO-A-94/23701
- WO-A-95/00126
- WO-A-95/03789
- WO-A-96/00295
- WO-A-98/08947
- LIN-SHU LIU ET AL.: "controlled release of interleukin-2 for tumour immunotherapy using alginate/chitosan porous microspheres" JOURNAL OF CONTROLLED RELEASE, vol. 43, no. 1, 3 January 1997 (1997-01-03), pages 65-74, XP000632680 Amsterdam (NL)

## Description

### TECHNICAL FIELD OF THE INVENTION

This invention is related to improved methods of immunization of mammals to achieve cell-mediated and humoral immune responses.

### BACKGROUND OF THE INVENTION

Upon immunization, plasmid DNA encoding genes for viral and bacterial antigens can direct the synthesis of the antigens in a native form and effectively present it to the immune system. For instance, potent humoral and cell-mediated immune responses have been induced by HIV-1 DNA vaccination in rodents and non-human primates [1, 2]. The plasmids are typically administered as bolus injections into the muscle or through a gene gun.

Recent findings that DNA immunization can elicit immune responses to viral proteins and confer protective immunity against challenge of the virus in rodent models have stimulated strong interest in optimizing this strategy for developing HIV vaccines [3-5]. Early generations of HIV vaccines that rely on recombinant antigens or killed virus antigens formulated with adjuvants have failed to generate effective CTL responses. DNA immunization offers the advantage of expressing the antigen in its native form that may lead to optimal processing and presentation to antigen presenting cells for induction of both humoral and cellular immune responses.

Although vaccinia vectors are efficient means of delivering the foreign genes *in vivo,* lingering concerns over the safety of these vectors remain. While extensive and elegant research is being conducted to engineer safer viral vectors, non-viral vectors are being increasingly proposed as alternatives. Chief among them for HIV vaccination are direct injection of the plasmid DNA into the muscular or dermal tissues, and bombardment of DNA-coated gold particles through a high pressure gene gun. Unfortunately, transfection efficiency is typically low. Thus there is a need in the art for methods of delivery of DNA which will provide an improved immune response.

The use of cytokines to enhance an immune response to vaccines has attracted intense attention, particularly in the field of cancer immunotherapy. One approach is to introduce cytokine genes directly into the tumor cells [6-23]. The cytokines either enhance the presentation of antigens to T cells or provide additional co-stimulatory signals for T cell activation. In many cases, the locally secreted cytokines elicit an inflammatory reaction that leads to the rejection of the injected tumor cells. In some cases, these genetically-altered tumor cells can generate systemic immunity against subsequent challenge of parental tumor cells, and occasionally even against established micrometastases.

GM-CSF and TNF-α have been found to synergize with IL-12 to enhance induction of cytotoxic T lymphocytes against HIV-1 MN vaccine constructs [1]. In a separate study, co-delivery of IL-12 with HIV-1 vaccines in mice resulted in splenomegaly and reduced humoral response, while GM-CSF has the opposite effect [24]. Both cytokines stimulated antigen-specific T cell responses, with a dramatic increase in CTL response observed for the co-delivery of IL-12. There is a need in the art for additional methods by which cytokines can be delivered to mammals to stimulate immune responses.

### SUMMARY OF THE INVENTION

It is an object of the present invention to provide solid microspheres for immunization of mammals.

It is another object of the present invention to provide a method for making solid microspheres for immunization of mammals.

It is an object of the present invention to provide a medicament for immunizing mammals using solid microspheres.

These and other objects of the invention are achieved by providing a solid microsphere for use in conjunction with genetic immunization of a mammal. The microsphere comprises a coacervate of a polymeric cation and a polyanion; the polymeric cation can be, for example, selected from the group consisting of gelatin and chitosan. The cytokine is encapsulated in the microsphere.

According to another aspect of the invention a medicament is provided for immunizing a mammal to raise an immune response to an antigen. The medicament comprises:
a nucleic acid encoding an antigen and a solid microsphere. The microsphere typically comprises a coacervate of a polymeric cation, such as gelatin or chitosan, and a polyanion. The microsphere encapsulates the cytokine.

In another embodiment of the invention a method of forming solid microspheres for immunization of a mammal is provided. The method comprises the step of:
forming solid microspheres by coacervation of a polyanion and a polymeric cation, wherein the polymeric cation is selected from the group consisting of chitosan and gelatin, wherein said coacervation is done in the presence of a cytokine, whereby the cytokine is encapsulated in said solid microspheres.

### BRIEF DESCRIPTION OF THE DRAWINGS

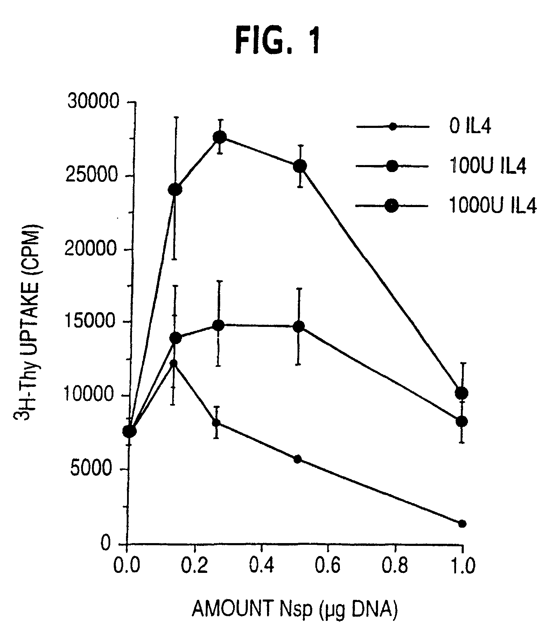

Figure 1. Proliferation of CT-4S cells in response to microsphere-delivered IL-4, DNA-gelatin microspheres were synthesized with different loading levels of murine IL-4 (denoted as 0, 100, and 1000U added to the coacervating reaction), were subsequently purified, and incubated with CT-4S cells. 30 hrs. after the addition of microspheres, the cells were labeled with ³H-thymidine, then radioactivity associated with the cells was measured 18 hrs later. Microsphere doses are indicated as total DNA. Data points are average of triplicates +/- s.d.

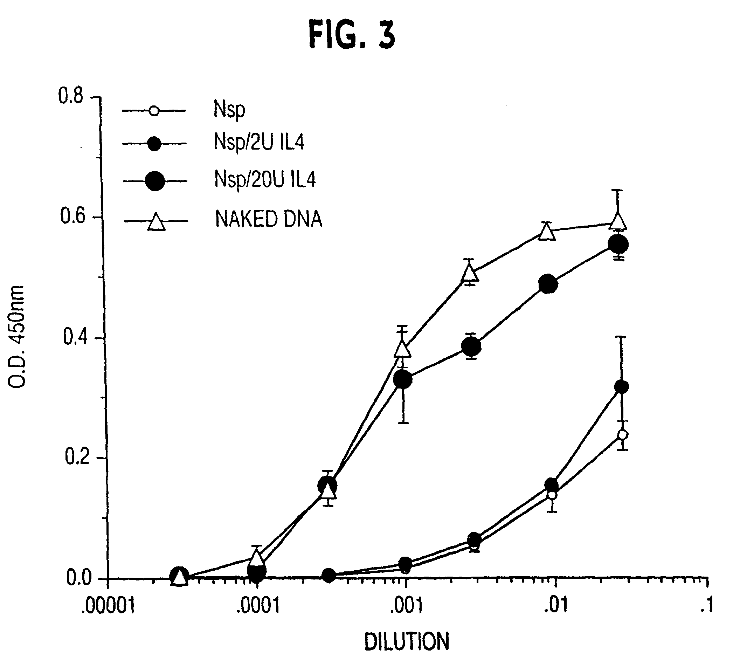

Figure 3. The anti-β-gal antibody response in mice injected with IL-4 given as microsphere-encapsulated vs. free form. Groups of 4 mice were injected i.m. with microspheres mixed with 20 U of free IL-4,. microspheres encapsulated with 20 U IL-4 per 1 mg p43-clacZ DNA, DNA, or DNA mixed with 20 U of free IL-4. Each group was vaccinated twice at four weeks intervals with 1 mg total DNA. At week 8, the sera from individual groups were pooled then assayed for total IgG anti-β-gal antibody. Data are average of triplicates +/- s.d.

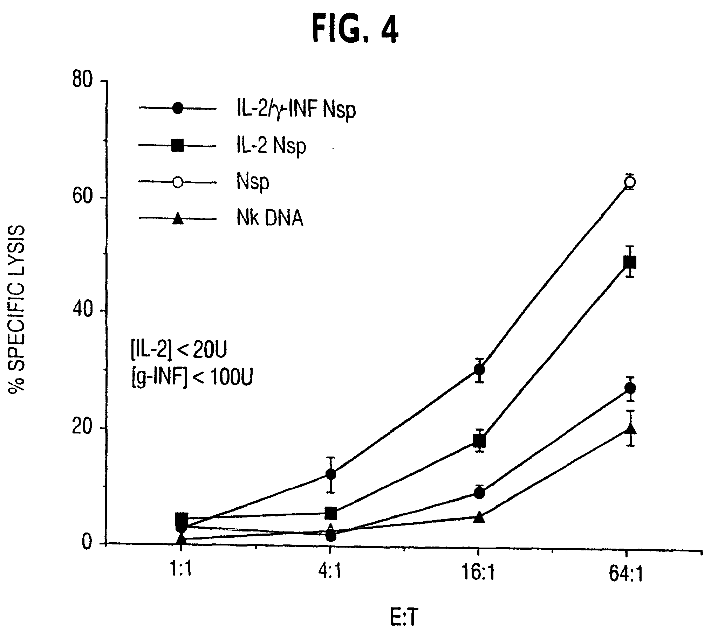

Figure 4. Potentiation of CTL responses using microsphere-encapsulated IL-2 and γ-INF. Groups of 4 mice were injected once i.m. with 2 mg of total p43-clacZ DNA either as microspheres, microspheres encapsulated with IL-2 (20 U per 1 mg p43-clacZ DNA), microspheres encapsulated with IL-2 and γ-INF (100 U per 1 mg pg 43-clacZ DNA), or 'naked' DNA. Standard CTL assays were carried out at week 4. Data are average +/- s,d.

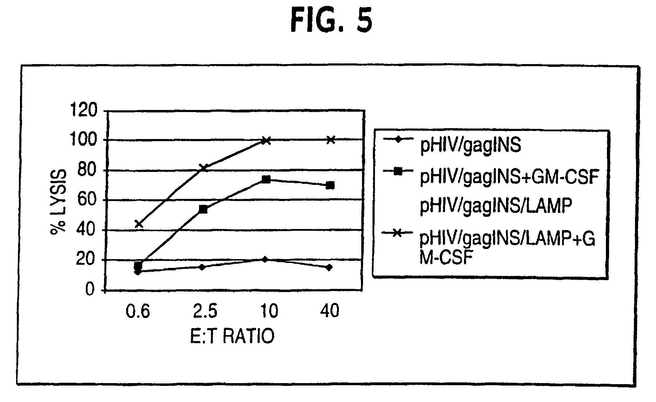

Figure 5. Antigen-specific cytolytic response when naked DNA was used to immunize animals in the presence and absence of microspheres encapsulating GM-CSF.

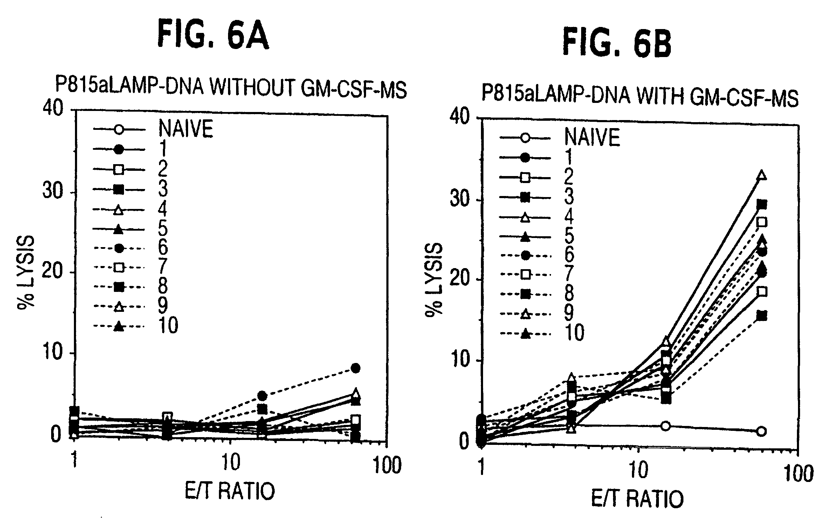

Figure 6. The gene encoding p815A was injected intradermally into mice with or without GM-CSF. Antigen-specific CTL of each individual mouse is shown. In Figure 6A, no cytokine microspheres were co-administered.

### DETAILED DESCRIPTION

The disclosure of prior U.S. patent applications Serial Nos. 08/265,966, and 08/657,913.

The inventors have found that microsphere controlled-release formulations of cytokines can maintain a high level of cytokines local to the vaccine site for days, providing co-stimulatory signals for infiltrating lymphocytes. The vaccine of the present invention thus comprises a DNA encoding an antigen and a microsphere-encapsulated cytokine. The two components can be administered together in a single formulation, or separately. The DNA can be in any form, encapsulated or naked, complexed or free. The DNA can be in the microspheres which provide the cytokines.

According to one embodiment of the invention microspheres can be synthesized by the salt-induced complex coacervation of a polyanion, such as chondroiton sulfate or nucleic acids, with a polycation, such as gelatin or chitosan. Other compositions are also possible for the microsphere, so long as a controlled release is effected. Cytokines are encapsulated in the microspheres to enhance the efficacy of genetic vaccination and to produce a desired immune response. The ultimate immune response elicited by the vaccine is influenced by the interaction between the specific and non-specific immune mechanisms. The type of immune cells attracted to the site of vaccination, for example, can determine the eventual antitumor immunity induced by a tumor-associated antigen. The repertoire of these cells is in turn governed by the temporal and spatial distribution of cytokines. By encapsulating the cytokine genes in the microspheres, the temporal and spatial distribution of the cytokines can be further altered. This can direct the immune response toward a specific immune arm, such as the Th1 or Th2 pathway. This strategy can be used, for example, to modulate the immune response against HIV infection by emphasizing the humoral or cellular arm.

Although any cytokine can be used which affects humoral or cell-mediated immune responses, a particularly preferred cytokine for use is GM-CSF. GM-CSF has been identified as a critical factor in inducing the differentiation of primitive hematopoietic precursors into dendritic cells. In vitro this differentiation requires at least six days of culture in the presence of GM-CSF, However, when administered as a bolus injection into the muscle, previous studies on tumor vaccines have shown that the cytokines would be cleared from the site of injection within hours. A microsphere controlled-release formulation can maintain a high level of cytokine local to the vaccine site for days, providing co-stimulatory signals for the infiltrating lymphocytes. GM-CSF and TNF-α have been found to synergize with IL-12 to enhance induction of cytotoxic T lymphocytes against HIV-1 MN vaccine constructs. In a separate study, co-delivery of IL-12 with HIV-1 vaccines in mice resulted in splenomegaly and reduced humoral response, while GM-CSF has the opposite effect. Both cytokines stimulated antigen-specific T cell responses, with an increase in CTL response observed for the co-delivery of IL-12.

Ligands can be conjugated to the microsphere to stimulate receptor-mediated endocytosis and potentially to target a cell/tissue. Lysosomolytic agents can be incorporated to promote escape of cytokines or DNA into cytoplasm. Other bioactive agents such as RNA, oligonucleotides, proteins, or multiple plasmids can be co-encapsulated. Relevant to vaccine application is that the protein antigen may also be co-encapsulated in the microsphere for potential augmentation of immune response through class II presentation, and as suggested by recent findings, as well as class I presentation when the microsphere is internalized by antigen presenting cells.

Bioavailability of administered nucleic acids can be improved because by targeting the nucleic acids to the endolysosomal pathway. This can be done, for example, by LAMP targeting. The prelysosomal/lysosomal localization signal from the LAMP (Lysosome-Associated Membrane Proteins) family of lysosomal membrane proteins can markedly enhance the immune response to the antigen. The LAMP molecules are type I transmembrane proteins containing a 24 amino acid transmembrane domain and an 11 amino acid cytoplasmic tail with a carboxyl-terminal YQTI sequence that is necessary and sufficient to target recombinant proteins through a vesicular pathway to lysosomes. Several studies have shown that LAMP molecules co-localize with MHC class II molecules in prelysosomal compartments. A chimeric DNA encoding an antigen protein with a targeting signal to the MHC II processing pathway thus can be used to enhance the presentation of the antigen to CD4+ helper T cells.

Microspheres according to the present invention are stable in plasma electrolytes, and can be lyophilized without loss of bioactivity. Hence the microcpheres can be handled more like conventional pharmaceutical formulations in terms of production, reproducibility, and storage. The rate and duration of cytokine delivery can be varied by changing the properties of the microspheres. Multiple cytokines can be readily used, and the tumor antigen to cytokine ratio can be varied easily.

Complex coacervation is a process of spontaneous phase separation that occurs when two oppositely charged polyelectrolytes are mixed in an aqueous solution. The electrostatic interaction between the two species of macromolecules results in the separation of a coacervate (polymer-rich phase) from the supernatant (polymer-poor phase). This phenomenon can be used to form microspheres and encapsulate a variety of compounds. The encapsulation process can be performed entirely in aqueous solution and at low temperatures, and has a good chance, therefore, of preserving the bioactivity of the encapsulant.

According to the present invention, gelatin or other polymeric cation having a similar charge density to gelatin, is used to complex with a polyanion, to form microspheres. Such polymeric cations Include, but are not limited to gelatin and chitosan. The source of gelatin is not thought to be critical; it can be from bovine, porcine, human, or other animal source. Typically the polymeric cation has a molecular weight of between 19,000-30,000. Poly-L-lysine or chitosan may be particularly useful as the polymeric cation of the present invention. Polyamino acids, synthetic or naturally occurring, can also be used, such as polylysine, poly-lysine-poly-arginine, polyarginine, protamine, spermine, spermidine, etc. Polysaccharides may also be used. Desirably sodium sulfate is used to induce the coacervation of polymeric cation and nucleic acids Ethanol can also be used at a concentration of about 40 to 60% to induce coacervation. Chondroitin sulfate can also be incorporated into the microsphere, which is especially beneficial if one desires other substances such as drugs and lysosomolytic agents to be incorporated in the microsphere. Typically the concentration of chondroitin sulfate is between about 0.005% and 0.1%.

The size of the microspheres is less than 5 microns. More preferred are microspheres of less than 3 microns, and even more preffered are microspheres which are less than 2, 1, 0-5, and 0.1 microns. While size can be effected by the conditions of coacervation and the size of the component polyanion and polycation microspheres of the desired size can also be size selected using a technique which separates the microspheres on the basis of size.

Targeting ligands, if desired, can be directly bound to the surface of the microsphere or can be indirectly attached using a "bridge" or spacer". Because of the amino groups provided by the lysine groups of the gelatin, the surface of the microspheres can be easily derivatized for the direct coupling of targeting moieties. For example, carbo-diimides can be used as a derivatizing agent. Alternatively, spacers (linking molecules and derivatizing moieties on targeting ligands) such as avidin-biotin can be used to indirectly couple targeting ligands to the microspheres. Biotinylated antibodies and/or other biotinylated ligands can be coupled to the avidin-coated microsphere surface efficiently because of the high affinity of biotin (kₐ∼10¹⁵ M⁻¹) for avidin (Hazuda, et al., 1990, Processing of precursor interleukin 1 beta and inflammatory disease, *J. Biol. Chem.,* **265:**6318-22; Wilchek, et al., 1990, Introduction to avidin-biotin technology, *Methods In Enzymology,* **184:**5-13). Orientation-selective attachment of IgGs can be achieved by biotinylating the antibody at the oligosaccharide groups found on the F_{c} portion (O'Shannessy, et al., 1984, A novel procedure for labeling immunoglobulins by conjugation to oligosaccharides moieties, *Immunol. Lett.,* **8:**273-277). This design helps to preserve the total number of available binding sites and renders the attached antibodies less immunogenic to F_{c} receptor-bearing cells such as macrophages. Spacers other than the avidin-biotin bridge can also be used, as are known in the art. For example, Staphylococcal protein A can be coated on the microspheres for binding the F_{c} portions of immunoglobulin molecules to the microspheres.

Cross-linking of linking molecules or targeting ligands to the microsphere is used to promote the stability of the microsphere as well as to covalently affix the linking molecule or targeting ligand to the microsphere. The degree of cross-linking directly affects the rate of nucleic acids released from the microspheres. Cross-linking can be accomplished using glutaraldehyde, carbodiimides such as EDC (1-ethyl-3-(3-dimethylaminopropyl)-carbodiimide, DCC (N.N'-dicyclohexylcarbodiimide), carboxyls (peptide bond) linkage, bis (sulfosuccinimidyl) suberate, dimethylsuberimidate, etc.

Targeting ligands according to the present invention are any molecules which bind to specific types of cells in the body. These may be any type of molecule for which a cellular receptor exists. Preferably the cellular receptors are expressed on specific cell types only. Examples of targeting ligands which may be used are hormones, antibodies, cell-adhesion molecules, saccharides, drugs which bind to cell receptors, and neurotransmitters.

The microspheres of the present invention have good loading properties.

Coacervation according to the present invention typically involves the interaction of polymeric cations and polycations. Because this process depends on the interaction of the positively charged polymeric cations and the negatively charged polyanions, such as nucleic acids, it can be considered as a complex coacervation process. However, sodium sulfate (or ethanol) induces the coacervation reaction by inducing a phase transition, and therefore it could also be considered as a simple coacervation reaction. Nucleic acids can be present in the coacervation mixture at a concentration of between 1 ng/ml to 500 µg/ml. Desirably the nucleic acids are at least about 2-3 kb in length. Sodium sulfate may be present at between 7 and 43 mM. Gelatin or other polymeric cation can be present at between about 2 and 7% in the coacervation mixture.

An attractive microsphere delivery system requires a delicate balance among factors such as the simplicity of preparation, cost effectiveness, nucleic acids loading level, controlled release ability, storage stability, and immunogenicity of the components. The microspheres described here may offer advantages compared to other particulate delivery systems, including the liposomal system. The problems of instability, low loading level, and controlled release ability are better resolved with the polymeric microsphere systems. Gelatin has received increasing biologic use ranging from surgical tissue adhesive (Weinschelbaum, et al., 1992, Surgical treatment of acute type A dissecting aneurysm with preservation of the native aortic valve and use of biologic glue. Follow-up to 6 years, *J. Thorac. Cardiovasc. Surg.,* **130:**369-74) to quantitative immunohistochemical assays (Izumi, et al., 1990, Novel gelatin particle agglutination test for serodiagnosis of leprosy in the field, J. *Clinical Microbiol.,* **28:**525-9) and as drug delivery vehicle (Tabata, et al., 1991, Effects of recombinant alpha-interferon-gelatin conjugate on *in vivo* murine tumor cell growth, *Cancer Res.,* **51:**5532-8), due to its biocompatibility and enzymatic degradability *in vivo*. Compared to other synthetic polymeric systems, such as the extensively studied polylactic/polyglycolic copolymers, the mild conditions of microsphere formulation are appealing. Unlike the solvent evaporation and hot-melt techniques used to formulate synthetic polymeric microspheres, complex coacervation requires neither contact with organic solvents nor heat. It is also particularly suitable for encapsulating bio-macromolecules, such as cytokines and nucleic acids not only through passive solvent capturing but also by direct charge-charge interactions.

Unlike viral vectors, which cannot deliver genes larger than 10 kb, the microsphere delivery system of the present invention does not have such size limitations. Nucleic acid molecules of greater than about 2 kb can be used, and nucleic acid molecules even greater than 10 kb may be used. Typically the nucleic acid will be greater than 300 bases, and typically geater than 0.5, 1, 2, 5, or 10 kb. Typically the nucleic acid molecule will be less than 200, 100, or 50 kb.

In general, the range of possible targets is dependent on the route of injection, *e.g.,* intravenous or intraarterial, subcutaneous, intra-peritoneal, intrathecal, etc. For systemic injections, the specificity of this delivery system is affected by the accessibility of the target to blood borne microspheres, which in turn, is affected by the size range of the particles. Size of the particles is affected by temperature, component concentration, and pH in the coacervation mixture. The particles can also be size-fractionated, e.g., by sucrose gradient ultracentrifugation. Particles with size less than 150 nanometers can access the interstitial space by traversing through the fenestrations that line most blood vessels walls. Under such circumstances, the range of cells that can be targeted is extensive. An abbreviated list of cells that can be targeted includes the parenchymal cells of the liver sinusoids, the fibroblasts of the connective tissues, the cells in the Islets of Langerhans in the pancreas, the cardiac myocytes, the Chief and parietal cells of the intestine, osteocytes and chondrocytes in the bone, keratinocytes, nerve cells of the peripheral nervous system, epithelial cells of the kidney and lung, Sertoli cells of the testis, etc. The targets for particles with sizes greater than 0.2 microns will be confined largely to the vascular compartment. Here, the targetable cell types include erythrocytes, leukocytes (i.e. monocytes, macrophages, B and T lymphocytes, neutrophils, natural killer cells, progenitor cells, mast cells, eosinophils), platelets, and endothelial cells. It is, however, not necessary that microspheres containing the cytokine be taken up by cells. They can exert their beneficial effect from an extracellular location. Thus size of the particles is not critical for cytokine delivery.

The cytokine and the DNA need not be administered at precisely the same time or in the same vehicle. They can be formulated separately and administered successively, *i.e.,* within a few seconds or minutes. Alternatively it may be desirable that the administrations be separated in time by hours or even days. If the administrations occur within a period of less than 72 hours to the same individual, a co-adnmistration according to the present invention has occurred.

The above disclosure generally describes the present invention. A more complete understanding can be obtained by reference to the following specific examples which are provided herein for purposes of illustration only, and are not intended to limit the scope of the invention.

### EXAMPLE 1

*Immnnological responses of pCI-clacZ plasmid DNA vectors.* Anti β-gal immune responses elicited by two lacZ expression vectors differing primarily by the presence of the adeno-associated virus inverted terminal repeats (AAV-ITR) were evaluated. The insertion of the CMV-intronA-lacZ (CMV/lacZ) cassette of p43-clacZ into an expression vector lacking the AAV-ITR (pCI-neo) resulted in markedly diminished anti-β-gal antibody and CTL responded in mice immunized i.m. with 'naked' DNA. At high DNA dose (100 mg). the p43-clacZ vector still out-performed the pCI-clacZ vector, although the difference in responses were significantly smaller as compared to that observed at the low dose (1 mg). With the exception of the neomycin resistance gene (*'neo'*) on pCI-clacZ, the only difference between pCI-clacZ and p43-clacZ is the AAV-ITR flanking the CMV/lacZ cassette. Since the *neo* gene does not contribute to lacZ gene expression, and it has no known immunological consequences in studies where it has been employed, these results suggest that the observed differences in anti-β-gal immunological responses may be attributable to the presence of the AAV-ITR. *In vitro* transfection studies using Lipofectamine reagents carried out on 293 cells showed no differences in the gene expression levels between these two vectors (data not shown), suggesting that the differences in immune response was not related to the level of gene expression.

### EXAMPLE 2

*Immunological responses of pCI-clacZ plasmid DNA vectors.* Anti β-gal immune responses elicited by two lacZ expression vectors differing primarily by the presence of the adeno-associated virus inverted terminal repeats (AAV-ITR) were evaluated. The insertion of the CMV-intronA-lacZ (CMV/lacZ) cassette of p43-clacZ into an expression vector lacking the AAV-ITR (pCI-neo) resulted in markedly diminished anti-β-gal antibody and CTL responded in mice immunized i.m. with 'naked' DNA . At high DNA dose (100 mg). the p43-clacZ vector still out-performed the pCI-clacZ vector, although the difference in responses were significantly smaller as compared to that observed at the low dose (1 mg). With the exception of the neomycin resistance gene (*'neo'*) on pCI-clacZ, the only difference between pCI-clacZ and p43-clacZ is the AAV-ITR flanking the CMV/lacZ cassette. Since the *neo* gene does not contribute to lacZ gene expression, and it has no known immunological consequences in studies where it has been employed, these results suggest that the observed differences in anti-β-gal immunological responses may be attributable to the presence of the AAV-ITR. *In vitro* transfection studies using Lipofectamine reagents carried out on 293 cells showed no differences in the gene expression levels between these two vectors (data not shown), suggesting that the differences in immune response was not related to the level of gene expression.

### EXAMPLE 3

*Cytokine production lymphocytes from microsphere-vaccinated mice.* To further characterize the immunological response of mice vaccinated with microspheres containing different doses of IL-4, the lymphocytes were harvested, stimulated with β-gal protein, then analyzed for IL-4 and y-INF production. Standard ELISA assays carried out on 3-day cultured serum revealed that mice immunized with microspheres with increasing IL-4 content produced proportionately more IL-4 upon re-stimulation with antigens. In these serum, a dose-related decrease in γ-INF production was observed. Because the production of IL-4 is associated with actively proliferating Tₕ2 helper cells, while γ-INF production is associated with Tₕ1 helper cells, it is concluded that in mice vaccinated with microsphere IL-4, the Tₕ2 helper cells response was potentiated while Tₕ1 helper cell response was depressed. This result, in combination with a dose-related potentiation of anti-β-gal antibody and a concomitant inhibition of CTL responses, is consistent with an IL-4 mediated immunological switch form a Tₕ 1-weighed response to that of a Tₕ2.

### EXAMPLE 4

*The effects of IL-4 given as microsphere-encapsulated vs. free form.* The effectiveness of IL-4 in potentiating an antibody response was examined in mice vaccinated with microspheres containing IL-4 either as microencapsulated or free form. ELISA assays carried out on mouse serum at week 8 (after two immunizations) showed that those injected with microspheres mixed with free IL-4 failed to elicit an enhancement in anti-β-gal antibody response (Fig. 3). In contrast, mice vaccinated with microsphere-encapsulated IL-4 generated a marked enhancement of antibody response. Moreover, mice injected with 'naked' DNA in the presence of the same IL-4 dose also failed to generate an immune enhancement as compared to those vaccinated with DNA alone. The data suggest that the IL-4 dose employed in the above experiment was effective in potentiating an antibody response only as microsphere-encapsulated form.

### EXAMPLE 5

*Potentiation of the CTL response using microsphere-encapsulated IL-2 and γ-INF.* The feasibility of immune response modulation using microsphere co-delivered cytokines was further demonstrated by examining the effects of microsphere-encapsulated IL-2 and γ-INF on the CTL responses. Mice immunized with a single injection of microspheres containing p43-clacZ DNA alone (2 mg total DNA), with IL-2, or with IL-2 and γ-INF were examined for the generation of anti-β-gal CTL responses at week 4. Mice vaccinated with microsphere alone or naked DNA generated poor CTL response (Fig. 4). This was consistent with our previous studies which showed at least two or three immunizations were necessary for strong CTL responses. However, when IL-2 was included in the microspheres, an enhancement in CTL response was observed. The CTL response was potentiated synergistically when γ-INF was co-delivered with EL-2 in the microspheres. The inclusion of both IL-2 and γ-INF in microsphere improved the anti-β-gal CTL response from 25% lysis (at an E:T ratio of 64:1) to at least 65% with just a single immunization.

### EXAMPLE 6

To compare the LAMP-targeting effect, we tested t-PA LSS/HIVgagINS/LAMP vs HIVgagINS (naked DNA) with and without I g GM-CSF in microspheres. As shown in Figure 5, when E:T ratio is 10, animals immunized with the chimeric gene showed a HIV-1 gag-specific CTL response of 38.2% lysis compared with 21.4% for the gene without LAMP targeting. When 1 g of GM-CSF encapsulated in microspheres was also included in the vaccine, the corresponding CTL responses at the same E:T ratio were enhanced to 100% and 75.6% lysis, respectively. For the gene without LAMP targeting, the co-delivery of GMCSF encapsulated in microspheres could improve the HIV gag-specific CTL response from 21% lysis to 75.6% at an E:T ratio of 10, while with the LAMP targeting gene, the improvement was from 38.2% to 100%. This clearly demonstrates the benefit obtained using the co-delivery of sustained release cytokine encapsulated microspheres. Cloning procedures

The human tissue plasminogen activator (t-PA) leading signal sequence (LSS) was amplified by polymerase chain reaction (PCR) from the gnomic DNA of human 293 cells, using primers t-PA sense (5' AAG CTGG CTAGCC CACCATGGATGC AATGAAGA GAGGGCTC 3') and t-PA & c-myc antisence (5' CGGCC GCTCGAGCAGATC CTCTTCTGA GATGAGTTTTTGTTCGAGGGGCGGGACACAGGGATC 3'). The c-myc epitope was added to the antisense primer. The resulting PCR product was digested by Nhel and Xhol and cloned into pCDNA3. 1(-). The HIV-1 gag INS gene was amplified by PCR from plasmid HIV-1gagINS neo pCDNA3.1 with primers gagINS sense (5' TCTAGA CTCGAG ATGGGTGCGAGAGCGTCA 3') and gagINS antisense(5'GTGGTGGAATTCCTGTGACGAGGGGTCGTT 3'), and cloned into t-PA LSS& c-myc neo pCDNA3. 1(-) after digested by Xhol and EcoRI.

The mouse lysosomal associated membrane protein(LAMP-1) transmembrane domain and c-terminal tail(Tmct) was amplified by PCR from plasmid LAMP-1 neo pCDNA3.1, which contains LAMP-1 gene, using primers LAMP Tmct sense(5' TCTGCAGAATTCCTGATCCCCATCGCTGTG 3') and LAMP Tmct antisense(5' AAGCTTGGTACCCTAGATAGTCTGGTAGCC. The PCR product was cloned into t-PA LSS & c-myc/HIV-1 gagINS neo pCDNA3. 1(-) after digested by EcoRI and Kpnl, resulting our t-PA LSS/HIV-1 gag INS/LAMP neo pCDNA3.1(-) construct which contains the cytomegalovirus(CMV) immediate early promoter to drive expression of our chimeric gene.
Mice immunization procedures and CTL assay:
Mouse strain used: Mouse strain Balb/c(H2d) was obtained from Charles Rivers. DNA was purified by CsCI /ethidium bromide equilibrium centrifugation Balb/c mice were immunized intramuscularly two times, at two weeks intervals, with 10g of plasmid. DNA was dissolved in PBS at a concentration of 0.2mg/ml, and 251 (5g) was injected into each anterior tibial muscle with or without 1g GM-CSF in microparticles, using a 28-gauge needle. Three weeks after immunization, the mice were killed and their spleens and lymph nodes were harvested. Splenocytes were isolated by homogenizing the spleens and lymph nodes, and then passing the suspension through a nylon mesh followed by Ficoll gradient centrifugation. Splenocytes from naive mice were labeled with HIV-1 gag-specific MHC class I peptide and inactivated by psoralen treatment and UV exposure for APC cells. T cells were enriched by nonadherence to nylon wool columns and then incubated in the presence of IL-2 and APC cells for seven days. Stimulated splenocytes were then used as effectors in a standard 51Cr release assay for CTL activity.
Cytokine microspheres: GM-CSF containing microspheres were synthesized as previously described .

### EXAMPLE 7

The gene encoding p815A, an antigen specific to mastocytoma cells, was injected intradermally into DAB/2 mice with or without GM-CSF controlled-release microspheres. Figure 6A shows the antigen-specific CTL of each individual mouse (10 in a group). No significant CTL was observed, consistent with typical results in the literature when the DNA vaccine is given without any booster shots. Significant CTL could be observed in 10 out of 10 animals when the DNA was administered with controlled delivery of GM-CSF in microspheres.

### References

1. Ahlers, J.D., N. Dunlop, D.W. Alling, P.L. Nara, and J.A. Berzofsky, Cytokine-in-adjuvant steering of the immune response phenotype to HIV- 1 vaccine constructs. J Immuno, 1997. 158 (8): p. 3947.
2. Arthos, S.L., et al., Simian inmmunodefiency Virus DNA Vaccine Trial in Macaques. J Viro, 1996. 70: p. 3978.
3. Donnelly, J.J., J.B. Ulmer, and M.A. Liu, DNA vaccines. Life Sci, 1997. 60: p. 163.
4. Regby, M.A., et al., Comparative efficiency of feline immunodeficiency virus infection by DNA inoculation. AIDS Res Hum Retroviruses. 1997. 13 (5): p. 405.
5. Wilson, J.M., Vectors-shuttle vehicles for gene therapy. Clin Exp Immunol, 1997. 107 Suppl 1: p. 31.
6. Tepper, R., P. Pattengale, and P. Leder, Murine interleukin-4 displays potent anti-tumor activity in vivo. Cell, 1989. 57 (May 5): p. 503-512.
7. Watanabe, Y., K. Kuribayashi, S. Miyatake, K. Nishihara, E. Nakayama, T. Taniyama, and T. Sakata, Exogenous expression of mouse interferon cDNA in mouse neuroblastoma C1300 cells results in reduced tumorigenicity by augmented anti-tumor immunity. Proc Natl Acad Sci, 1989. 86 (December): p. 9456-9460.
8. Fearon, E., et al., Interleukin-2 production by tumor cells bypasses T helper function in the generation of an antitumor response. Cell, 1990. 60 (February 9): p. 397-403.
9. Gansbacher, B., R. Bannerji, B. Daniels, K. Zier, K. Cronin, and E. Gilboa, Retroviral vector-mediated interferon gene transfer into tumor cells generates potent and long lasting antitumor immunity. Cancer research, 1990. 50 (December 15): p. 7820-7825.
10. Golumbek, P.T., A.J. Lazenby, H.I. Levitsky, E.M. Jaffe, H. Karasuyama, M. Baker, and D.M. Pardoll, Treatment of established renal cancer by tumor cells engineered to secrete interleukin-4. Science, 1991. 254: p. 713-716.
11. Hock, II., M. Dorsch, T. Diamantstein, and T. Blankenstein, Interleukin 7 induces CD4 T cell-dependent tumor rejection. J Exp Med, 1991. 174 (December); p. 1291-1298.
12. Restifo, N., P. Speiss, S. Karp, J. Mule, and S. Rosenberg, A nonimmumogenic sarcoma transduced with the cDNA for interferon elicits T cells against the wild-type tumor: correlation with antigen presentation capability. J Exp Med, 1992. 175 (June): p. 1423-1431.
13. Asher, A., et al., Murine tumor cells transduced with the gene for tumor necrosis factor: evidence for paracrine immune effects of tumor necrosis factor against tumors. J of immunology, 1991. 9 (May 1): p. 3227-3234.
14. Cavallo, F., M. Geovarelli, A. Gulino, A. Vacca, A. Stoppacciaro, A. Modesti, and G. Forni, Role of neutrophils and CD4 T lymphocytes in the primary and memory response to nonimmumogenic murine mammary adenocarcinoma made immunogenic by IL-2 gene. J of Immunology, 1992. 11 (December 1): p. 3627-3635.
15. Colombo, M., G. Ferrari, A. Stoppacciaro, M. Parenza, M. Rodolfo, F. Mavilio, and G. Parmiani, Granulocyte colony-stimulating factor gene transfer suppresses tumorigenicity of a murine adenocarcinoma in vivo. J Exp Med, 1991. 173 (April): p. 889-897.
16. Colombo, M., L. Lombardi, A. Stoppacciaro, C. Melani, M. Parenza, B. Bottazzi, and G. Parmiani, Granulocyte colony-stimulating factor (G-CSF) gene transduction in murine adenocarcinoma drives neutrophil-mediated tumor inhibition in vivo of immunology. J. of Immunology, 1992. 149, No 1 (Julyl): p. 113-119.
17. Dranoff, G., et al., Vaccination with irradiated tumor cells engineered to secrete murine granulocyte-macrophage colony-stimulating factor stimulates potent, specific, and long-lasting anti-tumor immunity. Proc Nat'1 Acad Sci, 1993. 90 (April): p. 3539-3543.
18. Porgador, A., E. Tzehoval, E. Vadai, M. Feldman, and L. Eisenbach, Immunotherapy via gene therapy: comparison of the effects of tumor cells transduced with the interleukin-2, interleukin-6 or interferon genes. J Immunother, 1993. 14, No 3: p. 191-201.
19. Porgador, A., M. Feldman, and L. Eisenbach, Immunotherapy of tumor mesastasis via gene therapy. Nat Immum, 1994. 13: p. 113-130.
20. Patel, P., C. Flemming, S. Russell, S. Eccles, and M. Collins, Cytokine gene transfer as a therapeutic strategy. J of Immunotherapy, 1993. 14, No 4: p. 310-313.
21. Yanagihara, K., T. Seyama, and Y. Watanabe, Antitumor potential of interferon: retroviral expression of mouse interferon cDNA in two kinds of highly metastatic mouse tumor lines reduces their tumorigenicity. Nat Immun, 1994. 13: p. 102-112.
22. Zatloukal, K., et al., Elicitation of a systemic and protective anti-melanoma immune response by an IL-2 based vaccine. J. Immunology, 1995. 154: p. 3406.
23. Tahara, H., et al., Effective eradication of established murine tumors with IL-12 gene therapy using a polycistronic retroviral vector. J. Immunology, 1995. 154: p. 6466.
24. Kim, J.J., et al., In vivo engineering of a cellular immune response by coadministration of IL-12 expression vector with a DNA immunogen. J Immunol, 1997. 158: p. 816.

## Claims

1. A solid microsphere of less than 5 microns for use in genetic immunization of a mammal, comprising a polymeric cation selected from the group consisting of chitosan, gelatin, poly-L-lysine, polylysine, poly-lysine-poly-arginine, polyarginine, protamine, spermine and spermidine, wherein a cytokine is encapsulated in the microsphere, wherein the solid microsphere comprises no DNA.

2. The microsphere of claim 1 which comprises gelatin.

3. The microsphere of claim 1 which comprises chitosan.

4. The microsphere of claim 1 wherein a cell targeting ligand is attached to said microsphere.

5. The microsphere of claim 4 wherein the targeting ligand is covalently attached to said microsphere by means of glutaraldehyde cross-linking.

6. The microsphere of claim 1 wherein the cytokine is selected from the group consisting of GM-CSF, TNF-α, IL-12, IL-4, γ-IFN, and combinations thereof.

7. The microsphere of claim 1 further comprising an encapsulated antigen.

8. Use of a nucleic acid encoding an antigen and a solid microsphere comprising an encapsulated cytokine, said microsphere comprising no DNA in the manufacture of a medicament for immunizing a mammal to raise an immune response to an antigen.

9. The use of claim 8 wherein the microsphere comprises a polymeric cation selected from the group consisting of gelatin and chitosan.

10. The use of claim 8 wherein the medicament is for injection into a muscle.

11. The use of claim 8 wherein the medicament is for subcutaneous injection.

12. The use of claim 8 wherein the medicament is for bombardment of the microspheres from a high pressure gene gun.

13. A method of forming solid microspheres for immunization of a mammal, comprising the steps of:
forming solid microspheres by coacervation of a polyanion and a polymeric cation,
wherein the polymeric cation is selected from the group consisting of chitosan, gelatin, poly-L-lysine, polylysine, poly-lysine-poly-arginine, polyarginine, protamine, spermine and spermidine, wherein said coacervation is done in the presence of a cytokine, wherein the polyanion is not nucleic acid, whereby the cytokine is encapsulated in said solid microspheres.

14. The method of claim 13 further comprising the steps:
cross-linking a cell targeting ligand to the microspheres.

15. The method of claim 13 wherein the coacervation is performed in the presence of sodium sulfate.

16. The method of claim 13 wherein the polymeric cation is gelatin.

17. The method of claim 13 wherein the polymeric cation is chitosan.

18. The method of claim 13 wherein a targeting ligand is adhered to the surface of said microsphere, said targeting ligand being selected from the group consisting of antibodies, hormones, cell-adhesion molecules, saccharides, drugs which bind to cellular receptors and neurotransmitters.

19. The method of claim 16 wherein the gelatin is present at a concentration of about 2-7% in the step of coacervation.

20. The method of claim 13 wherein sodium sulfate is present in the step of coacervation at a concentration between 7 and 43 mM.

## Patentansprüche

1. Festes Mikrokügelchen von weniger als 5 Mikrometer zur Verwendung bei der genetischen Immunisierung eines Säugers, umfassend ein polymeres Kation, ausgewählt aus der Gruppe bestehend aus Chitosan, Gelatine, Poly-L-Lysin, Polylysin, Polylysin-Polyarginin, Polyarginin, Protamin, Spermin und Spermidin, wobei ein Zytokin in dem Mikrokügelchen eingebettet ist, wobei das feste Mikrokügelchen keine DNA umfaßt.

2. Mikrokügelchen nach Anspruch 1, welches Gelatine umfaßt.

3. Mikrokügelchen nach Anspruch 1, welches Chitosan umfaßt.

4. Mikrokügelchen nach Anspruch 1, wobei ein Zelltargeting-Ligand an das Mikrokügelchen gebunden ist.

5. Mikrokügelchen nach Anspruch 4, wobei der Targeting-Ligand mittels Glutaraldehyd-Verknüpfung kovalent an das Mikrokügelchen gebunden ist.

6. Mikrokügelchen nach Anspruch 1, wobei das Zytokin ausgewählt ist aus der Gruppe bestehend aus GM-CSF, TNF-α, IL-12, IL-4, γ-IFN und Kombinationen davon.

7. Mikrokügelchen nach Anspruch 1, welches ferner ein eingebettetes Antigen umfaßt.

8. Verwendung einer für ein Antigen codierenden Nukleinsäure und eines festen Mikrokügelchens, das ein eingebettetes Zytokin umfaßt, wobei das Mikrokügelchen keine DNA umfaßt, zur Herstellung eines Medikaments zum Immunisieren eines Säugers, um eine Immunreaktion auf ein Antigen hervorzurufen.

9. Verwendung nach Anspruch 8, wobei das Mikrokügelchen ein polymeres Kation umfaßt, ausgewählt aus der Gruppe bestehend aus Gelatine und Chitosan.

10. Verwendung nach Anspruch 8, wobei das Medikament zur Injektion in einen Muskel ist.

11. Verwendung nach Anspruch 8, wobei das Medikament zur subkutanen Injektion ist.

12. Verwendung nach Anspruch 8, wobei das Medikament zum Beschuß mit Mikrokügelchen aus einer Hochdruck-Genkanone ist.

13. Verfahren zur Bildung von festen Mikrokügelchen zur Immunisierung eines Säugers, umfassend die Schritte:
Bilden fester Mikrokügelchen durch Koazervierung eines Polyanions und eines polymeren Kations, wobei das polymere Kation ausgewählt ist aus der Gruppe bestehend aus Chitosan, Gelatine, Poly-L-Lysin, Polylysin, Polylysin-Polyarginin, Polyarginin, Protamin, Spermin und Spermidin, wobei die Koazervierung in Gegenwart eines Zytokins erfolgt, wobei das Polyanion keine Nukleinsäure ist, wodurch das Zytokin in die festen Mikrokügelchen eingebettet wird.

14. Verfahren nach Anspruch 13, welches ferner die Schritte umfaßt:
Verknüpfen eines Zelltargeting-Liganden mit den Mikrokügelchen.

15. Verfahren nach Anspruch 13, wobei die Koazervierung in Gegenwart von Natriumsulfat erfolgt.

16. Verfahren nach Anspruch 13, wobei das polymere Kation Gelatine ist.

17. Verfahren nach Anspruch 13, wobei das polymere Kation Chitosan ist.

18. Verfahren nach Anspruch 13, wobei ein Targeting-Ligand an die Oberfläche des Mikrokügelchens gebunden wird, wobei der Targeting-Ligand ausgewählt ist aus der Gruppe bestehend aus Antikörpern, Hormonen, Zelladhäsionsmolekülen, Sacchariden, Wirkstoffen, die an Zellrezeptoren binden, und Neurotransmittern.

19. Verfahren nach Anspruch 16, wobei die Gelatine in dem Koazervierungsschritt in einer Konzentration von etwa 2-7% vorliegt.

20. Verfahren nach Anspruch 13, wobei Natriumsulfat in dem Koazervierungsschritt in einer Konzentration zwischen 7 und 43 mM vorliegt.

## Revendications

1. Microsphère solide d'une taille inférieure à 5 µm utilisée pour une utilisation dans l'immunisation génétique d'un mammifère, comprenant un cation polymère choisi dans le groupe consistant en le chitosane, la gélatine, la poly-L-lysine, la polylysine, la polylysine-polyarginine, la polyarginine, la protamine, la spermine et la spermidine, dans laquelle une cytokine est encapsulée dans la microsphère, dans laquelle la microsphère solide ne comprend pas d'ADN.

2. Microsphère selon la Revendication 1 qui comprend de la gélatine.

3. Microsphère selon la Revendication 1 qui comprend du chitosane.

4. Microsphère selon la Revendication 1, dans laquelle un ligand de ciblage de cellules est fixé à ladite microsphère.

5. Microsphère selon la Revendication 4, dans laquelle le ligand de ciblage est fixé par une liaison covalente à ladite microsphère au moyen d'une réticulation au glutaraldéhyde.

6. Microsphère selon la Revendication 1, dans laquelle la cytokine est choisie dans le groupe consistant en GM-CSF, TNF-α, IL-12, IL-4, γ-IFN et leurs combinaisons.

7. Microsphère selon la Revendication 1 comprenant en outre un antigène encapsulé.

8. Utilisation d'un acide nucléique encodant un antigène et d'une microsphère solide comprenant une cytokine encapsulée, ladite microsphère ne comprenant pas d'ADN, dans la fabrication d'un médicament pour immuniser un mammifère pour augmenter une réponse immunitaire à un antigène.

9. Utilisation selon la Revendication 8, dans laquelle la microsphère comprend un cation polymère choisi dans le groupe consistant en la gélatine et le chitosane.

10. Utilisation selon la Revendication 8, dans laquelle le médicament est destiné à être injecté dans un muscle.

11. Utilisation selon la Revendication 8, dans laquelle le médicament est destiné à une injection sous-cutanée.

12. Utilisation selon la Revendication 8, dans laquelle le médicament est destiné à un bombardement de microsphères par un canon à gènes à haute pression.

13. Procédé de fabrication de microsphères solides pour l'immunisation d'un mammifère, comprenant les étapes de :
fabrication de microsphères solides par coacervation d'un polyanion et d'un cation polymère, dans lequel le cation polymère est choisi dans le groupe consistant en le chitosane, la gélatine, la poly-L-lysine, la polylysine, la polylysine-polyarginine, la polyarginine, la protamine, la spermine et la spermidine, dans lequel ladite coacervation est effectuée en présence d'une cytokine, dans lequel le polyanion n'est pas un acide nucléique, selon lequel la cytokine est encapsulée dans lesdites microsphères solides.

14. Procédé selon la Revendication 13 comprenant en outre les étapes de :
réticulation d'un ligand de ciblage de cellules sur les microsphères.

15. Procédé selon la Revendication 13, dans lequel la coacervation est effectuée en présence de sulfate de sodium.

16. Procédé selon la Revendication 13, dans lequel le cation polymère est la gélatine.

17. Procédé selon la Revendication 13, dans lequel le cation polymère est le chitosane.

18. Procédé selon la Revendication 13, dans lequel un ligand de ciblage est en état d'adhérence avec la surface de ladite microsphère, ledit ligand de ciblage étant choisi dans le groupe consistant en les anticorps, les hormones, les molécules d'adhérence aux cellules, les saccharides, les médicaments qui se lient aux récepteurs cellulaires et aux neurotransmetteurs.

19. Procédé selon la Revendication 16, dans lequel la gélatine est présente à une concentration d'environ 2 à 7% dans l'étape de coacervation.

20. Procédé selon la Revendication 13, dans lequel le sulfate de sodium est présent dans l'étape de coacervation à une concentration comprise entre 7 et 43 mM.
